**Europäisches Patentamt**

**European Patent Office**

**Office européen des brevets**

(19)

(11) Publication number : **0 384 390 B1**

# (12) EUROPEAN PATENT SPECIFICATION

(45) Date of publication of patent specification :
23.06.93 Bulletin 93/25

(51) Int. Cl.⁵ : **C07C 249/04, B01J 21/06**

(21) Application number : **90103233.4**

(22) Date of filing : **20.02.90**

(54) Catalytic process for the preparation of oximes.

(30) Priority : **21.02.89 IT 1949989**

(43) Date of publication of application :
**29.08.90 Bulletin 90/35**

(45) Publication of the grant of the patent :
**23.06.93 Bulletin 93/25**

(84) Designated Contracting States :
**BE DE ES FR GB NL**

(56) References cited :
**EP-A- 0 267 362**
**EP-A- 0 311 983**
**EP-A- 0 347 926**
**CHEMICAL ABSTRACTS, vol. 84, no. 17, 26th**
**April 1976, page 497, abstract no. 121316a,**
**Columbus, Ohio, US; & JP-A-50 151 839**

(73) Proprietor : **MONTEDIPE S.r.l.**
**31, Foro Buonaparte**
**I-20121 Milan (IT)**

(72) Inventor : **Petrini, Guido**
**11, Via Pasubio**
**I-28066 Galliate, Novara (IT)**
Inventor : **Padovan, Mario**
**1, Via Villa Mirabello**
**I-20125 Milan (IT)**
Inventor : **Genoni, Fausto**
**20, Via Verdi**
**I-21017 Samarate, Varese (IT)**
Inventor : **Leofanti, Giuseppe**
**43, Via Firenze**
**I-20010 Canegrate, Milan (IT)**
Inventor : **Roffia, Paolo**
**25, Via Valletta**
**I-21047 Saronno, Varese (IT)**
Inventor : **Cesana, Alberto**
**6, Via Torino**
**I-20048 Carate Brianza, Milan (IT)**

(74) Representative : **Weinhold, Peter, Dr. et al**
**Patentanwälte Dr. V. Schmied-Kowarzik**
**Dipl.-Ing. G. Dannenberg Dr. P. Weinhold Dr. D.**
**Gudel Dipl.-Ing. S. Schubert Dr. P. Barz**
**Siegfriedstrasse 8**
**W-8000 München 40 (DE)**

EP 0 384 390 B1

## Description

EP-A-208,311 describes the preparation of cyclohexanone-oxime in the liquid phase from cyclohexanone, ammonia and hydrogen peroxide in the presence of a catalyst consisting of a crystalline compound having a zeolitic structure. The obtainment of said zeolitic structure, however, necessitates the treatment of silicon compounds and titanium compounds with organic compounds known as templating agents, in particular tetraalkylammonium hydroxides or salts thereof, which can be synthesized only with extreme difficulty.

EP-A-299,430 and 311,983 suggest to use, as the source of silicon and/or titanium, solid amorphous materials but nevertheless, in order to obtain from said amorphous materials a crystalline structure which is catalytically active, the presence of templating agents cannot be dispensed with.

The preparation of said crystalline structure requires very long residence times and the use of high temperatures and pressures. Furthermore, it is necessary to subject the catalyst to complex post-treatments. Subsequently it was found (see IT-A-21076 A/88) that the synthesis of the oximes may also be promoted by titanium- and silicon-based catalysts which do not exhibit the above zeolitic structure.

It has now, surprisingly, been found that the synthesis of oximes, starting from carbonyl compounds, may also be promoted by catalysts based on titanium only, or containing silicon at the most in very low concentrations (traces), which catalysts may be prepared without the use of templating agents in a very simple way and within a very short time.

In its broadest aspect the present invention relates to a catalytic process for the preparation of oximes by reaction, in the liquid phase, of the corresponding carbonyl compounds with $NH_3$ and $H_2O_2$ (ammoximation). Said process is characterized in that the catalyst is selected from solid compositions substantially consisting of titanium and oxygen, chemically combined with each other, said compositions being preferably selected from:

a) crystalline compositions, particularly rutile, anatase and brookite;

b) amorphous compositions, i.e. compositions characterized by an XR-diffractogram, obtained by X-ray diffraction using the $K_\alpha$ radiation of copper, which shows, in the $(2\vartheta)$ range from 10° to 65°, a smooth-trend line (halo), typical for amorphous solids;

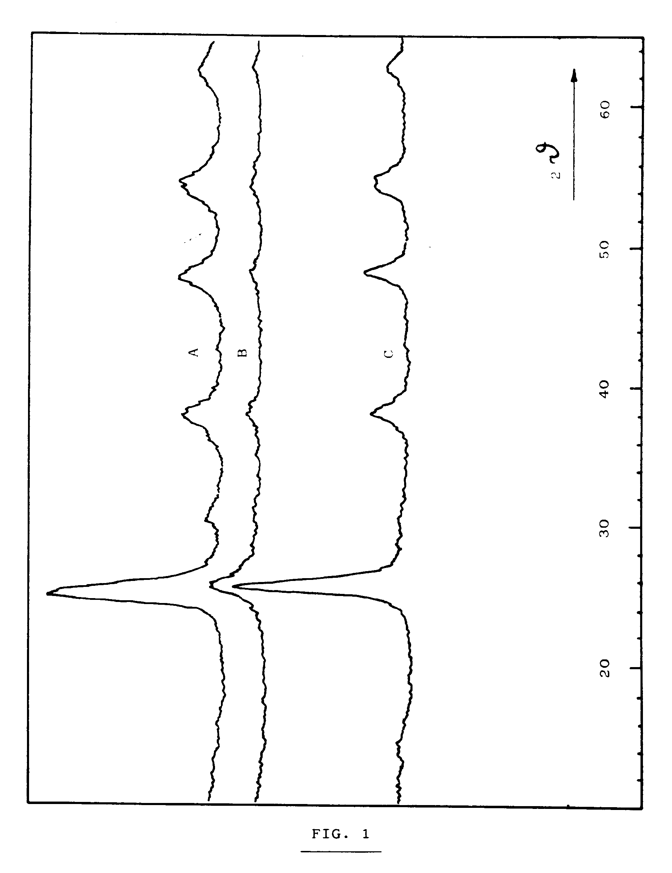

c) mixed compositions, i.e. compositions characterized by an XR-diffractogram which, in addition to said halo, also shows the reflections of rutile and/or anatase and/or brookite (a typical example thereof is represented by the diffractogram of figure 1);

d) mixtures of compositions according to a), b) and c) above.

In said compositions, as already mentioned, silicon is absent or present at the most in trace amounts. The surface area of said compositions preferably ranges from 10 to 1000 $m^2/g$, most preferred from 100 to 800 $m^2/g$.

The present catalysts have also been employed in a continuous process for many hours without any sign of exhaustion, with yields equal to and sometimes higher than the ones obtained in the discontinuous tests, and they proved to be very active not only in the case of the ammoximation of aldehydes and ketones, but also in the case of other organic syntheses such as, e.g., the synthesis of N,N-dialkylhydroxylamines described in EP-A-314,147.

Aldehydes that may catalytically be converted into oximes in general are aldehydes of formula $R_1CHO$, wherein $R_1$ is selected from alkyl, cycloalkyl, aryl or heterocyclic groups (containing one or more of O, N and S atoms in the ring) having from 1 to 18 carbon atoms.

The term "alkyl group" also includes arylalkyl and cycloalkyl-alkyl groups as well as alkyl groups substituted with heterocyclic groups.

"Cycloalkyl group" also covers alkyl-cycloalkyl and aryl-cycloalkyl groups as well as cycloalkyl groups substituted with heterocyclic groups.

"Heterocyclic groups" includes alkyl-, cycloalkyl- or aryl-substituted heterocyclic groups.

Specific examples of the (basic) groups mentioned above are

$C_{1-12}$-, particularly $C_{1-6}$-alkyl such as methyl, ethyl, n-propyl, i-propyl, n-butyl, i-butyl, sec-butyl and tert.-butyl and pentyl;

$C_{4-12}$-cycloalkyl, such as cyclopentyl and cyclohexyl;

$C_{6-12}$-aryl, such as phenyl, naphthyl and biphenylyl;

$C_{5-10}$-heterocyclic groups such as pyrrolyl, pyridinyl, pyrimidinyl, furanyl, pyranyl, thienyl and the completely or partially saturated equivalents thereof.

The ketones that can catalytically be converted into oximes generally are ketones of formula $R_2$-CO-$R_3$, wherein $R_2$ and $R_3$, the same or different from each other, have the same meaning as $R_1$ and can be linked at their ends, thus forming a carbocyclic or heterocyclic ring.

Particularly preferred carbonyl compounds are acetone, cyclohexanone, methyl-ethyl-ketone, acetophenone, benzophenone, tert-butyl-cyclohexanone, cyclo-dodecanone, enanthic aldehyde (1-heptanal) and benzaldehyde.

The catalyst can be prepared starting from various titanium and silicon sources, according to methods which are known for the preparation of heterogeneous catalysts or, more generally, of oxides.

Without being limitative in any way of the scope of the invention, a few possible procedures are given below:

a) The catalyst of the present invention can be prepared by hydrolysis of alcoholic solutions containing titanium alcoholates, and, optionally, small amounts of tetraalkylsilicate (see, for example: Journal of Catalysis 102, pages 249-251 (1986)).

The hydrolysis may be carried out by blowing an inert gas, saturated with water vapor (steam), into the alcoholic solution, possibly heated at a temperature lower than or equal to the boiling temperature.

b) Alternatively, the catalyst may be obtained from aqueous solutions of hydrosoluble titanium compounds by precipitation with a base (for example ammonium hydroxide); see, for instance, Journal of Catalysis 105 (1987), pages 511-520.

c) The method according to b) may also be applied in the case of aqueous solutions of soluble trivalent titanium compounds. In this case, after precipitation, the product is subjected to oxidation, for example by blowing air into the suspension or by calcination after filtration and washing; see, for instance, Journal of Colloid and Interface Science; Vol. 101(2), October 1984, page 369.

d) A volatile titanium compound may be subjected to combustion (see, for example, Kirk-Othmer, Encyclopedia of Chemical Technology, 3rd edition, vol.23, page 145).

After the preparation the dried catalyst can be used directly for the ammoximation or it may be calcined in a stream of air or of another gas, or under vacuum, at a temperature ranging from 50° to 800°C.

As exemplary sources of titanium the following may be mentioned:
- alkyl titanates and, particularly, tetraisopropyl titanate and/or di-isopropyl-bis(triethanolamine)titanate;
- titanium halides and, particularly, titanium tetrachloride ($TiCl_4$) and/or titanium trichloride ($TiCl_3$);
- combinations thereof and their equivalents.

Generally, the conversion of the carbonyl compound into the oxime is carried out in the liquid phase at temperatures from 25° to 100°C, preferably of from 40° to 90°C, and even more preferred of from 60° to 90°C.

Tests carried out at 15°C generally did not result in satisfactory results.

The reaction may be conducted at atmospheric pressure or at slightly higher than atmospheric pressure, in order to keep dissolved in the reaction medium an amount of $NH_3$ which is at least commensurate with the requirements of the synthesis. The catalyst may be provided on a fixed bed, in particular a trickle-bed, or may be finely dispersed in the reaction medium, provided the reactor surfaces can withstand hydrogen peroxide. If the reaction is performed discontinuously it is advisable to use from 0.1 to 50 parts by weight, preferably from 1 to 20 parts, of catalyst per 100 parts of carbonyl compound.

If the reaction is performed continuously, it is recommended to operate at a space velocity of from 0.1 to 200 kg/hour of carbonyl compound per kg of catalyst. The molar ratio $H_2O_2$/carbonyl compound in general ranges from 0.3 to 2.5, preferably from 0.5 to 1.3, $H_2O_2$ being calculated as 100% pure hydrogen peroxide, dilution water being excluded. The molar ratio $NH_3/H_2O_2$ generally is equal to or greater than 1, preferably greater than 1.5, since otherwise parallel reactions might occur.

The reaction medium may consist of water and/or organic solvents.

Particularly good results may be obtained by using as solvent tert-butanol and/or cyclohexanol, optionally in admixture with dioxane or toluene. The molar ratio of tert-butanol (and/or cyclohexanol) to carbonyl compound preferably ranges from 0.1 to 100. At the end of the reaction the oxime may be separated in different ways, for example by means of extraction with suitable solvents such as, for instance, benzene, toluene or the ketone (or aldehyde) employed as starting material, whereby a hydrophobic organic phase and an aqueous phase are formed.

Oxime and unreacted carbonyl compound are present in the organic layer. The aqueous layer, containing the excess $NH_3$ as well as traces or carbonyl compound and oxime, may suitably be recycled to the reaction.

Alternatively, the extraction may be conducted simultaneously with the synthesis by operating in a two-phase system. A suitable system may be prepared by using a couple of solvents of different characteristics, for example tert-butanol (hydrophilic) and toluene (hydrophobic). In case the ammoximation is to be conducted continuously, it is recommended to maintain the space velocity in the range of from 0.1 to 200 kg/h of carbonyl compound (preferably from 2 to 200 kg/h) per kg of pure catalyst, binders excluded, and to feed the carbonyl compound in admixture with the organic solvent, for instance tert-butanol (and/or cyclohexanol). For the ammoximation the technique of percolation on a catalytic bed, also called "trickle-bed" technology,may conveniently be used. In this case the catalyst preferably is in the form of pellets, rings, optionally polylobated extrudates etc. One of the alternatives to percolation is the continuous reaction in a suspended bed and under stirring.

In this case it is convenient to feed the reactants through dipping pipes, immersed beneath the level of the liquid. Obviously, one or more reactants may be introduced portionwise during the reaction, for instance through a series of inlet or feeding points, thus achieving a better control of the reaction.

The following examples are given in order to illustrate the present invention without, however, being a limitation of possible embodiments thereof.

## EXAMPLE 1

A solution of 300 g of tetraisopropyl-orthotitanate in 300 ml of anhydrous isopropanol was mixed, at room temperature under vigorous stirring and by dripping (dripping rate: 5 ml/minute), with 300 ml of $H_2O$.

Stirring was continued for 4 hours, whereafter the product was filtered, washed with distilled $H_2O$ and finally dried for 16 hours at 120°C. Thereupon the product was calcined for 2 hours at 200°C (heating rate of 1°C/minute).

Thereby a catalyst with a surface area of 185 $m^2/g$ was obtained. The corresponding XR-diffractogram, shown in figure 1, is marked by the letter 'A' and shows the presence of the more intensive reflection of anatase (d = 0.352 nm; $2\vartheta$ = 25.3°); see card JCPDS-21-1272.

## EXAMPLE 2

A glass reactor, equipped with stirrer and heating jacket, was previously pressurized with an inert gas (nitrogen).

Into the reactor were then introduced 15.06 g of the catalyst powder obtained according to example 1. Thereupon there were added 21 g of water (1.17 mol), 25 g of tert-butanol (0.34 mol) and 4 g of ammonia (0.24 mol). The resulting mixture was subjected to stirring and then there were added 10.34 g of cyclohexanone (0.105 mol), thus forming a bi-phasic (solid/liquid) system which was kept homogeneous by means of vigorous stirring. The temperature was raised to 80°C by feeding into the heating jacket of the reactor a thermostatic liquid.

Thereupon an aqueous 33% b.w. solution of hydrogen peroxide was introduced into the reactor by means of a metering pump. During the heating, the pressure rose slightly above atmospheric pressure. The addition of $H_2O_2$ was continued for 5 hours, the total amount of added $H_2O_2$ being 11.33 g (0.096 mol). During the addition the pressure inside the autoclave dropped.

To the suspension thus obtained there was added, after cooling, diethyl ether and the resulting mixture was stirred for a few minutes. The aqueous and ethereal phases were then separated from the catalyst by means of filtration whereafter the liquid phases were separated in a separatory funnel. Gas-chromatographic analysis revealed a cyclohexanone conversion of 72.8% and a selectivity to oxime of 88.4%.

The oxime yield (with respect to $H_2O_2$) was 61.4%.

All data and results are recorded in Table 1.

## EXAMPLE 3

200 g of tetraisopropyl-orthotitanate were placed in a flask provided with reflux cooler, thermometer, magnetic stirrer and a dipping pipe for bubbling in a hydrous inert gas (nitrogen).

Then there were added 150 ml of anhydrous isopropanol and into the resulting solution, heated to 60°C, there was injected a stream of an inert gas, previously passed through a scrubber, said gas containing $H_2O$ heated up to 60°C. A total of 50 ml of water was thus introduced into the reactor in the form of steam during a period of 20 hours. The product obtained at the end of the hydrolysis was filtered, washed with anhydrous isopropanol and dried for 15 hours at 120°C. The product was finally calcined at 200°C for 2 hours.

There was obtained a catalytic powder with a surface area of 478 $m^2/g$. The corresponding XR-diffractogram, shown in figure 1, is marked by the letter "B".

## EXAMPLE 4

To a solution of 754 g of $TiCl_3$ (in the form of a 15% b.w. aqueous solution) in 3.5 litres of distilled $H_2O$ were added, under vigorous stirring and at room temperature, 560 ml of a 15% b.w. aqueous solution of ammonia, until reaching neutrality (pH=7.1). The black precipitate of the thus formed titanous hydrate was then oxidized to the white (titanic) form by bubbling air through the suspension.

The white titanium hydroxide was then filtered, washed until complete disappearance of the Cl⁻ ions from the washing water, and finally dried for 48 hours at a temperature of 120°C. One portion of the thus obtained solid was calcined for 6 hours at 300°C. Thereby a catalytic powder having a surface area of 244 $m^2$/g was obtained. The corresponding XR-diffractogram, shown in figure 1, is indicated by the letter "C".

EXAMPLES 5 AND 6

Example 2 was repeated, varying the catalyst as indicated in Table 1 which also shows the results of the corresponding ammoximation.

TABLE 1

| EXAMPLE | 2 | 5 | 6 |
|---|---|---|---|
| Catalyst (type) | from ex.1 | from ex.3 | from ex.4 |
| catalyst (g) | 15.06 | 15.02 | 7.51 |
| $TiO_2$ (%) | 100 | 100 | 100 |
| Surface area ($m^2$/g) | 185 | 478 | 224 |
| XR-diffraction | A | B | C |
| ketone conversion (%) | 72.8 | 74.5 | 69.6 |
| selectivity of ketone to oxime (%) | 88.4 | 87.4 | 84.0 |
| oxime yield (based on $H_2O_2$) (%) | 61.4 | 61.6 | 55.4 |

**Claims**

1. Catalytic process for the preparation of oximes by reaction, in the liquid phase, of the corresponding carbonyl compounds with $NH_3$ and $H_2O_2$ (ammoximation), wherein the catalyst is selected from solid compositions substantially consisting of titanium and oxygen chemically combined with each other.

2. Process according to claim 1, wherein said solid compositions are selected from:
   a) crystalline compositions, particularly rutile, anatase and brookite;
   b) amorphous compositions, i.e. compositions characterized by an XR-diffractogram, obtained by means of X-ray diffraction using the $K_\alpha$ radiation of copper, which shows, in the $(2\vartheta)$ range from 10° to 65°, a smooth-trend line (halo) typical for amorphous solids;
   c) mixed compositions, i.e. compositions characterized by an XR-diffractogram which, in addition to said halo, also shows the reflections of rutile and/or anatase and/or brookite;
   d) mixtures of compositions according to a), b) and c).

3. Process according to anyone of claims 1 and 2, wherein said compositions have a surface area from 10 to 1000 $m^2$/g, particularly from 100 to 800 $m^2$/g.

4. Process according to anyone of the preceding claims, wherein said compositions are obtained by using, as source of titanium, compounds selected from:
   - alkyl titanates, in particular tetraisopropyl titanate and/or di-isopropyl-bis(triethanolamine)titanate;
   - titanium halides, in particular titanium tetrachloride and titanium trichloride;

5

- combinations and equivalents thereof.

5. Process according to anyone of the preceding claims, wherein said compositions are obtained by using, as source of titanium, a compound selected from $TiCl_4$, tetraisopropyltitanate and/or di(isopropyl)-bis(triethanolamine)-titanate.

6. Process according to anyone of the preceding claims, wherein the catalytic compositions are obtained by hydrolysis of alcoholic solutions of a titanium alcoholate or from aqueous solutions of hydrosoluble titanium compounds by precipitation with a base, in particular ammonium hydroxide ($NH_4OH$).

7. Process according to anyone of the preceding claims, wherein the catalytic compositions, before being used, are calcined at a temperature of from 50° to 800°C.

8. Process according to anyone of the preceding claims, wherein the carbonyl compound is an aldehyde of formula $R_1CHO$, wherein $R_1$ is selected from alkyl, cycloalkyl, aryl or heterocyclic groups having from 1 to 18 carbon atoms, or is a ketone of formula $R_2\text{-}CO\text{-}R_3$, wherein $R_2$ and $R_3$, the same or different from each other, have the same meaning as $R_1$ and, additionally, may be combined to form a carbocyclic or heterocyclic ring.

9. Process according to anyone of the preceding claims, wherein the carbonyl compound is selected from acetone, cyclohexanone, methylethyl-ketone, acetophenone, benzophenone, tert.-butyl-cyclohexanone, cyclo-dodecanone, enanthic aldehyde and benzaldehyde.

10. A solid catalytic composition, particularly suitable for the preparation of oximes by ammoximation of the corresponding carbonyl compounds, substantially consisting of titanium and oxygen, chemically combined with each other, and being characterized by an XR-diffractogram, obtained by means of X-ray diffraction using the $K_\alpha$ radiation of copper, which, in the $(2\vartheta)$ range from 10° to 65°, shows a smooth-trend line (halo) typical for amorphous solids and, optionally, the reflections of rutile and/or anatase and/or brookite.

## Patentansprüche

1. Katalytisches Verfahren zur Herstellung von Oximen durch Umsetzung der entsprechenden Carbonylverbindungen in flüssiger Phase mit $NH_3$ und $H_2O_2$ (Ammoximierung), worin der Katalyator aus festen Zusammensetzungen ausgewählt ist, die im wesentlichen aus chemisch miteinander kombiniertem Titan und Sauerstoff bestehen.

2. Verfahren nach Anspruch 1, worin die festen Zusammensetzungen ausgewählt sind aus:
   a) kristallinen Zusammensetzungen, insbesondere Rutil, Anatas und Brookit;
   b) amorphen Zusammensetzungen, d.h. Zusammensetzugen, die durch ein Röntgen-Diffraktogramm, erhalten durch Röntgenbeugung unter Verwendung der $K_\alpha$-Strahlung von Kupfer, gekennzeichnet sind, das im $(2\vartheta)$-Bereich von 10° bis 65° eine diffuse Linie (Halo) zeigt, die für amorphe Feststoffe typisch ist;
   c) gemischten Zusammensetzungen, d.h. Zusammensetzungen, die durch ein Röntgen-Diffraktogramm gekennzeichnet sind, das zusätzlich zum Halo auch die Reflektionen von Rutil und/oder Anatas und/oder Brookit zeigt;
   d) Mischungen von Zusammensetzungen gemäß a), b) und c).

3. Verfahren nach irgendeinem der Ansprüche 1 und 2, worin die Zusammensetzungen eine Oberfläche von 10 bis 1000 m²/g, insbesondere von 100 bis 800 m²/g, haben.

4. Verfahren nach irgendeinem der vorhergehenden Ansprüche, worin die Zusammensetzungen erhalten werden, indem man als Titanquelle Verbindungen verwendet, die ausgewählt sind aus:
   - Alkyltitanaten, insbesondere Tetraisopropyltitanat und/oder Diisopropyl-bis-(triethanolamin)-titanat;
   - Titanhalogeniden, insbesondere Titantetrachlorid und Titantrichlorid;
   - Kombinationen und Äquivalenten derselben.

5. Verfahren nach irgendeinem der vorhergehenden Ansprüche, worin die Zusammensetzungen erhalten werden, indem man als Titanquelle eine Verbindung, ausgewählt aus $TiCl_4$, Tetraisopropyltitanat und/oder

Di-(isopropyl)-bis-(triethanolamin)-titanat verwendet.

**6.** Verfahren nach irgendeinem der vorhergehenden Ansprüche, worin die katalytischen Zusammensetzungen durch Hydrolyse alkoholischer Lösungen eines Titanalkoholates oder aus wäßrigen Lösungen wasserlöslicher Titanverbindungen durch Ausfällen mit einer Base, insbesondere Ammoniumhydroxid ($NH_4OH$), erhalten werden.

**7.** Verfahren nach irgendeinem der vorhergehenden Ansprüche, worin die katalytischen Zusammensetzungen vor der Verwendung bei einer Temperatur von 50 bis 800°C calciniert werden.

**8.** Verfahren nach irgendeinem der vorhergehenden Ansprüche, worin die Carbonylverbindung ein Aldehyd der Formel $R_1CHO$, worin $R_1$ ausgewählt ist aus Alkyl, Cycloalkyl, Aryl oder heterocyclischen Gruppen mit 1 bis 18 Kohlenstoffatomen, oder ein Keton der Formel $R_2$-$CO$-$R_3$ ist, worin $R_2$ und $R_3$, die gleich oder voneinander verschieden sein können, die gleiche Bedeutung wie $R_1$ haben und zusätzlich zur Bildung eines carbocyclischen oder heterocyclischen Ringes kombiniert werden können.

**9.** Verfahren nach irgendeinem der vorhergehenden Ansprüche, worin die Carbonylverbindung aus Aceton, Cyclohexanon, Methylethylketon, Acetophenon, Benzophenon, tert-Butylcyclohexanon, Cyclododecanon, Önanthsäurealdehyd und Benzaldehyd ausgewählt ist.

**10.** Feste katalytische Zusammensetzung, insbesondere geeignet zur Herstellung von Oximen durch Ammoximierung der entsprechenden Carbonylverbindungen, die im wesentlichen aus chemisch miteinander kombiniertem Titan und Sauerstoff bestehen und durch ein Röntgen-Diffraktogramm, erhalten durch Röntgenbeugung unter Verwendung durch $K_\alpha$-Strahlung von Kupfer, gekennzeichnet sind, das im $(2\vartheta)$-Bereich von 10° bis 65° eine diffuse Linie (Halo), die für amorphe Feststoffe typisch ist, und fakultativ die Reflektionen von Rutil und/oder Anatas und/oder Brookit zeigt.

**Revendications**

**1.** Procédé catalytique pour la préparation d'oximes par réaction, en phase liquide, des composés carbonylés correspondants avec de l'ammoniac $NH_3$ et de l'eau oxygénée $H_2O_2$ (ammoximation), dans lequel le catalyseur est choisi parmi des compositions solides, constituées essentiellement de titane et d'oxygène combinés chimiquement l'un à l'autre.

**2.** Procédé selon la revendication 1, dans lequel lesdites compositions solides sont choisies parmi :
a) des compositions cristallines, en particulier le rutile, l'anatase et la brookite,
b) des compositions amorphes, c'est-à-dire des compositions caractérisées par un diagramme de diffraction des rayons X, que l'on obtient par diffraction des rayons X en utilisant la raie $K\alpha$ du cuivre, présentant, dans l'intervalle $(2\theta)$ de 10° à 65°, une tache diffuse (halo), typique de solides amorphes,
c) des compositions mixtes, c'est-à-dire des compositions caractérisées par un diagramme de diffraction des rayons X qui présente , en plus dudit halo, les réflexions du rutile et/ou de l'anatase et/ou de la brookite,
d) des mélanges de compositions selon a), b) et c).

**3.** Procédé selon la revendication 1 ou 2, dans lequel lesdites compositions ont une surface spécifique de 10 à 1000 $m^2$/g, en particulier de 100 à 800 $m^2$/g.

**4.** Procédé selon l'une quelconque des revendications précédentes, dans lequel lesdites compositions sont obtenues par utilisation, en tant que source de titane, de. composés choisis parmi :
- les titanates d'alkyle, en particulier le titanate de tétraisopropyle et/ou le bis(triéthanolamine)titanate de diisopropyle,
- les halogénures de titane, en particulier le tétrachlorure de titane et le trichlorure de titane,
- leurs combinaisons et équivalents.

**5.** Procédé selon l'une quelconque des revendications précédentes, dans lequel lesdites compositions sont obtenues par utilisation de, en tant que source de titane, un composé choisi parmi $TiCl_4$, le titanate de tétraisopropyle et/ou le bis(triéthanolamine)titanate de diisopropyle.

6. Procédé selon l'une quelconque des revendications précédentes, dans lequel les compositions catalytiques sont obtenues par hydrolyse de solutions alcooliques d'un alcoolate de titane, ou à partir de solutions aqueuses de dérivés du titane hydrosolubles, par précipitation avec une base, en particulier l'hydroxyde d'ammonium ($NH_4OH$).

7. Procédé selon l'une quelconque des revendications précédentes, dans lequel les compositions catalytiques sont calcinées à une température de 50° à 800°C, avant d'être utilisées.

8. Procédé selon l'une quelconque des revendications précédentes, dans lequel le dérivé carbonylé est un aldéhyde de formule $R_1CHO$, dans laquelle $R_1$ est choisi parmi les groupes alkyle, cycloalkyle, aryle ou hétérocycliques, ayant de 1 à 18 atomes de carbone, ou est une cétone de formule $R_2\text{-}CO\text{-}R_3$, dans laquelle $R_2$ et $R_3$ sont identiques ou différents et ont chacun la même signification que $R_1$, et, en plus, peuvent être combinés pour former un noyau carbocyclique ou hétérocyclique.

9. Procédé selon l'une quelconque des revendications précédentes, dans lequel le composé carbonylé est choisi parmi l'acétone, la cyclohexanone, la méthyléthylcétone, l'acétophénone, la benzophénone, la tert.-butyl-cyclohexanone, la cyclododécanone l'aldéhyde énanthique et le benzaldéhyde.

10. Composition catalytique solide, convenant en particulier à la préparation d'oximes par ammoximation des composés carbonylés correspondants, constituée essentiellement de titane et d'oxygène, combinés chimiquement l'un à l'autre, et caractérisée par un diagramme de diffraction des rayons X, que l'on obtient par diffraction des rayons X en utilisant la raie $K\alpha$ du cuivre, présentant, dans l'intervalle ($2\theta$) allant de 10° à 65°, une tache diffuse (halo), typique de solides amorphes, et, éventuellement, les réflexions du rutile et/ou de l'anatase et/ou de la brookite.

FIG. 1